# EUROPÄISCHE PATENTSCHRIFT

(11) **EP 0 494 868 B1**
(45) Veröffentlichungstag und Bekanntmachung des Hinweises auf die Patenterteilung: **12.10.1994**
(21) Anmeldenummer: 90910719.5
(22) Anmeldetag: 03.07.1990
(51) Int. Cl.: C09J 4/06, C08F 251/00

(54) **SELBSTKLEBENDES LEITENDES ELASTISCHES GEL**
SELF-ADHESIVE CONDUCTIVE ELASTIC GEL
GEL ELASTIQUE CONDUCTEUR AUTO-ADHESIF

(43) Veröffentlichungstag der Anmeldung: 22.07.1992
(73) Patentinhaber: ZIMMER ELEKTROMEDIZIN GmbH, D-89231 Neu-Ulm (DE)
(72) Erfinder: Schmid, Walter, W-7914 Pfaffenhofen/Roth (DE)
(74) Vertreter: Fay, Hermann, Dipl.-Phys. Dr.
(86) Internationale Anmeldenummer: EP9001064
(87) Internationale Veröffentlichungsnummer: WO9201026

(56) Entgegenhaltungen:
- FR-A- 2 305 452
- WPIL, File Supplier, AN-82-34384e, Derwent Publications Ltd, London, GB; & JP-A-57 049 431
- WPIL, File Supplier, AN-84-122956, Derwent Publications Ltd, London, GB; & JP-A-59 058 764

## Beschreibung

Die Erfindung betrifft ein selbstklebendes leitendes elastisches Gel, das inbesondere zur Herstellung von Körperelektroden verwendet wird.

Aus dem Dokument DE-A-36 09 137 sind Filme aus elektrisch leitfähigen Polymeren bekannt, die sich zur Verwendung als Elektrodenmaterialien eignen. Diese Filme enthalten 0,1 bis 50 Gewichtsteile eines wasserlöslichen, im wesentlichen säuregruppenfreien Polymeren, welcher in Form von Celluloseäther und Cellulose vorliegen kann. Diese Filme werden durch elektrochemische Polymerisation der Monomeren an flächigen Elektroden in wässrigen Elektrolyt-Lösungsmitteln hergestellt. Anschließend werden die erhaltenen Filme von der Elektrode, auf der sie abgeschieden wurden, abgelöst.

Im Dokument DE-A-27 40 270 wird eine Elektrode für medizinische Zwecke, insbesondere zur Aufnahme von Elektrokardiogrammen, beschrieben, welche als die Haut kontaktierendes leitendes selbstklebendes Gel ein hydrophiles Polysaccharid, wie Karaya-Gum, aufweist.

Im Dokument FR-A-2 305 425 wird die Herstellung wasserabsorbierender Harze beschrieben, die erhältlich sind durch Polymerisation von mindestens einem Polysaccharid, mindestens einem Monomer mit einer Doppelbindung und einem Vernetzer. Als Monomerkomponente kann dabei ein Monomer des Acrylsäuretyps und als Vernetzerkomponente das Salz einer schwachen Säure und eines mehrwertigen Metalls verwendet werden. In den im Dokument aufgeführten Beispielen sind folgende Komponenten zu finden: Maisstärke, Acrylsäure und Natriumacrylat, ein Vernetzer und Ammonium-Cer-nitrat als Katalysator. Natriumacrylat und Ammonium-Cer-nitrat können aufgrund ihres ionischen Charakters als Elektrolyten fungieren. Die Produkte können vor dem Trocknungs- und Pulverisierungsvorgang entweder als weiße viskose Suspension oder als weißer elastischer Festkörper anfallen.

Die bekannten Gele sind relativ kostspielig und müssen in technisch aufwendiger Weise in heißem Zustand in die Körperelektroden eingebracht oder auf diese aufgebracht werden. Darüber hinaus läßt ihr Klebevermögen an der Haut, insbesondere nach mehrmaligem Gebrauch, zu wünschen übrig.

Die Erfindung hat sich daher die Aufgabe gestellt, unter Beseitigung der Nachteile der bekannten Gele für Körperelektroden ein Gel zu schaffen, das einfach und preiswert herzustellen ist, eine bessere Klebekraft, auch nach mehrmaligem Gebrauch, als die bekannten Gele besitzt und nicht in heißem Zustand in eine Körperelektrode eingebracht oder in beliebiger Schicht auf eine Körperelektrode aufgebracht werden muß.

Ein nach der Erfindung lösendes Gel der eingangs genannten Art ist dadurch gekennzeichnet, daß es erhältlich ist durch Polymerisation von acrylsäuregruppenhaltigen Monomeren oder acrylsäuregruppenhaltigen Monomermischungen unter Bildung von hautverträglichen Polymeren oder Copolymeren in einer einen Elektrolyten enthaltenden Lösung eines hautverträglichen Stärkematerials, wobei die Lösung 5 bis 50 Gewichtsteile des Stärkematerials, 10 bis 50 Gewichtsteile des bzw. der Monomeren und 3 bis 10 Gewichtsteile des Elektrolyten enthält.

Die Unteransprüche betreffen bevorzugte Ausführungsformen des erfindungsgemäßen Gels.

Die Erfindung betrifft außerdem die Verwendung des erfindungsgemäßen Gels.

Die Erfindung beruht auf der überraschenden Feststellung daß dann, wenn in einer Lösung eines Stärkematerials ein acrylsäuregruppenhaltiges Monomeres oder eine acrylsäuregruppenhaltige Monomermischung, die zu einem hautverträglichen Polymeren oder Copolymeren führt, polymerisiert wird, ein Gel erhalten wird, das ein besseres Klebevermögen an der Haut auch bei mehrmaligem Gebrauch besitzt als die bisher bekannten Gele.

Ferner ist es erfindungsgemäß möglich, die Lösung des Stärkematerials vor der Polymerisation des bzw. der Monomeren in eine Körperelektrode oder auf diese in kaltem Zustand aufzubringen und dann die Polymerisation zur Ausbildung des Gels durchzuführen.

Die zur Herstellung des erfindungsgemäßen Gels verwendete Lösung eines Stärkematerials ist vorzugsweise eine wässrige Lösung und die zur Polymerisation eingesetzten Monomeren sind ebenfalls wasserlöslich.

Als Stärkematerialien werden bevorzugterweise Amylopektine mit Molekulargewichten zwischen 300000 und 200000 verwendet. Die Stärkematerialien können auch in verschiedener Weise substituiert sein.

Als Monomere, die in der Lösung des Stärkematerials polymerisiert werden, werden acrylsäuregruppenhaltige Monomere oder acrylsäuregruppenhaltige Monomermischungen verwendet, welche sich vorzugsweise durch freie Radikale erzeugende Substanzen oder durch Strahlung, wie UV-Strahlung, Elektronenstrahlung, β- oder UV-Strahlung polymerisieren lassen.

Bevorzugt eingesetzt werden als Monomere Acryl- oder Methacrylsäure oder Ester davon, wie Allylacrylat. Man kann auch Vorläufer für derartige polymerisierbare Monomere in der zu polymerisierenden Lösung einsetzen, wie beispielsweise β-Hydroxypropionsäure, die sich beim Erhitzen in Acrylsäure umwandelt, welches dann polymerisiert.

Vorzugsweise enthält die Lösung des Stärkematerials in welcher die genannten Monomeren polymerisiert werden, ein Feuchthaltemittel, das auch gleichzeitig als Weichmacher sowie zur Erhöhung der Klebrigkeit dienen kann. Für diesen Zweck besonders geeignete Substanzen sind mehrwertige Alkohole, wie Diole, Triole und Polyole, insbesondere Propantriol sowie Glyzerin.

Desweiteren kann der Lösung des Stärkematerials ein den pH-Wert erhöhendes Mittel, wie Alkanolamine, zugesetzt werden.

Wie bereits erwähnt, kann die Polymerisation gegenüber freie Radikale liefernde Substanzen initiiert werden. Als derartige, eine chemische Initiierung der Polymerisation bewirkende Substanzen kommen die üblicherweise eingesetzten Verbindungen, wie Perverbindungen, in Frage.

Die Polymerisation kann auch durch Strahlung initiiert werden, wobei in diesem Falle der Lösung des Stärkematerials vorzugsweise ein an sich bekannter Fotoinitiator verwendet wird, dessen Auswahl von der verwendeten Strahlung abhängt.

Das erfindungsgemäße Gel kann nach seiner Fertigstellung in entsprechender Abmessung in die an sich bekannten Körperelektroden als die Haut kontaktierende leitende selbstklebende Schicht eingesetzt werden, in zweckmäßiger Weise erfolgt jedoch die Polymerisation der Monomeren in der Lösung des Stärkematerials in situ in oder auf der Elektrode. Zu diesem Zweck wird eine Lösung des Stärkematerials hergestellt, die neben einem Elektrolylten sowie ggfs. einem Feuchthaltemittel und einer den pH-Wert erhöhenden Substanz je nach Art der Polymerisation eine freie Radikale liefernde Verbindung oder einen Fotoinitiator enthält, worauf zu dieser Lösung eine Lösung des bzw. der zu polymerisierenden Monomeren gegeben wird. Das erhaltene Gemisch wird dann in eine Elektrode ein- bzw. auf eine Elektrode aufgebracht und entweder durch Einwirkung von Wärme oder Strahlung polymerisiert.

Als Elektrolyte werden erfindungsgemäß vorzugsweise hautverträgliche Salze verwendet, beispielsweise Alkalichloride, wie Kaliumchlorid.

In zweckmäßiger Weise enthält die Lösung, in welcher die Polymerisation erfolgt, außer dem Stärkematerial, dem bzw. den Monomeren und dem Elektrolyten noch 1 bis 2 Gewichtsteile eines Initiators sowie 10 bis 50 Gewichtsteile eines Feuchthaltemittels. Ggfs. kann das Wasser einer wässrigen Lösung vollständig durch das Feuchthaltemittel, wie Glyzerin, ersetzt sein.
durch Auswahl und Konzentration der verwendeten Stärkematerialien und der zu polymerisierenden acrylsäuregruppenhaltigen Monomeren sowie des Polymerisationsgrades, bis zu welchem die Polymerisation durchgeführt wird, lassen sich die gewünschten Eigenschaften der erfindungsgemäßen Gele maßschneidern, insbesondere ihre Konsistenz und ihr Klebevermögen.

Die folgenden Beispiele erläutern die Erfindung.

### Beispiel 1

15 Gewichtsteile destilliertes Wasser und 35 Gewichtsteile Propandiol werden miteinander vermischt. Dann werden 3 Gewichtsteile Kaliumchlorid in der erhaltenen Mischung aufgelöst, Anschließend werden 5 Gewichtsteile Amylopektin zugesetzt und bei einer Temperatur von 90°C unter Rühren aufgelöst.

Nachdem das Amylopektin in Lösung gegangen ist, wird die Mischung auf 40°C abgekühlt, worauf 33,7 Gewichtsteile β-Hydroxypropionsäure zugegeben werden. Dann wird die Temperatur unter Rühren auf 50°C erhöht, was eine Umwandlung der Hydroxypropionsäure in Acrylsäure zur Folge hat. Anschließend wird die Mischung auf 25°C abgekühlt, worauf 8 Gewichtsteile Triethanolamin unter langsamen Rühren zugegeben werden. Dann werden 2 Gewichtsteile eines acrylierten Benzophenons als Initiator unter Rühren zugemischt. Die Mischung wird bei einer Temperatur von 20°C langsam während einer Zeitspanne von 3 Stunden unter Lichtausschluß gerührt. Anschließend wird die Mischung auf eine silikonisierte Folie in einer Schichtdicke von 2 mm aufgetragen und mit einer UV-Strahlung mit einer Wellenlänge von 300 nm in einem Abstand von 250 mm während einer Zeitspanne von 30 Sekunden polymerisiert.

Der elektrische Leitwert (Impedanz) AC-Z bei 10 Hz des erhaltenen Gels beträgt 240 Ohm. Der pH-Wert wird zu 4,8 ermittelt und eine Hautfreundlichkeit des Gels festgestellt.

### Beispiel 2

100 Gewichtsteile destilliertes Wasser und 20 Gewichtsteile Propantriol werden vermischt. 10 Gewichtsteile Kaliumchlorid werden in der Mischung aufgelöst. Anschließend werden 20 Gewichtsteile Amylopektin unter Rühren zugesetzt und auf 90°C erhitzt, bis sich das Amylopektin aufgelöst hat. Dann wird die erhaltene Lösung (Lösung 1) auf 20°C abgekühlt. 30 Gewichtsteile Propantriol und 30 Gewichtsteile der Lösung 1 werden miteinander vermischt. Anschließend werden 0,2 Gewichtsteile Ethylenglykoldimethacrylat und 10 Gewichtsteile Triethanolamin unter Rühren zugesetzt bei einer konstanten Temperatur von 20°C. Anschließend werden unter langsamem Rühren 15 Gewichtsteile Acrylsäure während einer Zeitspannen von 20 Minuten eingetropft. Bei einer Temperatur von 50°C sowie unter Rühren werden 0,5 Gewichtsteile Kaliumpersulfat in gelöster Form zugegeben, worauf 1 Stunde polymerisiert wird.

Das erhaltene Gel besitzt einen elektrischen Leitwert (Impedanz) AC-Z bei 10 Hz von 260 Ohm. Sein pH-Wert wird zu 4,5 ermittelt, außerdem wird festgestellt, daß das Gel hautfreundlich ist.

## Patentansprüche

1. Selbstklebendes leitendes elastisches Gel auf der Basis eines wasserlöslichen Polymeren und wasserlöslicher Monomere, dadurch gekennzeichnet, daß es erhältlich ist durch Polymerisation von acrylsäuregruppenhaltigen Monomeren oder acrylsäuregruppenhaltigen Monomermischungen unter Bildung von hautverträglichen Polymeren oder Copolymeren in einer einen Elektrolyten enthaltenden Lösung eines hautverträglichen Stärkematerials, wobei die Lösung 5 bis 50 Gewichtsteile des Stärkematerials, 10 bis 50 Gewichtsteile des bzw. der Monomeren und 3 bis 10 Gewichtsteile des Elektrolyten enthält.

2. Gel nach Anspruch 1, dadurch gekennzeichnet, daß es erhältlich ist unter Verwendung von Amylopektin als Stärkematerial.

3. Gel nach den Ansprüchen 1 und 2, dadurch gekennzeichnet, daß es erhältlich ist unter Verwendung von Acryl- oder Methacrylsäure oder Estern davon oder deren Vorläufer.

4. Gel nach den Ansprüchen 1 bis 3, dadurch gekennzeichnet, daß es erhältlich ist unter Verwendung einer Lösung des Stärkematerials, die ein Feuchthaltemittel enthält.

5. Gel nach den Ansprüchen 1 bis 4, dadurch gekennzeichnet, daß es erhältlich ist unter Verwendung einer Lösung des Stärkematerials, das eine den pH-Wert erhöhende Substanz enthält.

6. Gel nach den Ansprüchen 1 bis 5, dadurch gekennzeichnet, daß es erhältlich ist unter Verwendung einer Lösung des Stärkematerials, die einen freie Radikale liefernden Initiator oder einen Fotoinitiator für die Polymerisation der Monomeren enthält.

7. Gel nach den Ansprüchen 1 bis 6, dadurch gekennzeichnet, daß es erhältlich ist unter Verwendung einer Lösung des Stärkematerials, in welcher ein hautverträgliches Salz als Elektrolyt enthalten ist.

8. Gel nach den Ansprüchen 1 bis 7, dadurch gekennzeichnet, daß es erhältlich ist unter Verwendung einer wässrigen Lösung, welche außer dem Stärkematerial, dem bzw. den Monomeren und dem Elektrolyten noch 1 bis 2 Gewichtsteile eines freie Radikale liefernden Initiators enthält.

9. Verwendung eines Gels nach den Ansprüchen 1 bis 8 zur Herstellung von Körperelektroden.

## Claims

1. A self-adhesive conductive elastic gel based on a water-soluble polymer and water-soluble monomers, characterised in that it is obtainable by polymerisation of acrylic acid group-bearing monomers or acrylic acid group-bearing monomer mixtures, with the formation of skin-compatible polymers or copolymers, in an electrolyte-containing solution of a skin-compatible starch material, wherein the solution contains from 5 to 50 parts by weight of the starch material, from 10 to 50 parts by weight of the monomer or monomers, and from 3 to 10 parts by weight of the electrolyte.

2. A gel according to claim 1 characterised in that it is obtainable using amylopectin as the starch material.

3. A gel according to claims 1 and 2 characterised in that it is obtainable using acrylic or methacrylic acid or esters thereof or their precursors.

4. A gel according to claims 1 to 3 characterised in that it is obtainable using a solution of the starch material, which contains a moistening agent.

5. A gel according to claims 1 to 4 characterised in that it is obtainable using a solution of the starch material which contains a substance for increasing the pH-value.

6. A gel according to claims 1 to 5 characterised in that it is obtainable using a solution of the starch material, which contains a free radical-supplying initiator or a photoinitiator for polymerisation of the monomers.

7. A gel according to claims 1 to 6 characterised in that it is obtainable using a solution of the starch material, in which a skin-compatible salt is contained as electrolyte.

8. A gel according to claims 1 to 7 characterised in that it is obtainable using an aqueous solution which, besides the starch material, the monomer or monomers and the electrolyte, also contains from 1 to 2 parts by weight of a free radical-supplying initiator.

9. Use of a gel according to claims 1 to 8 for the production of body electrodes.

## Revendications

1. Gel élastique conducteur autocollant sur la base d'un polymère soluble dans l'eau et de monomères solubles dans l'eau, caractérisé en ce qu'il peut être obtenu par polymérisation de monomères contenant des groupes d'acide acrylique ou de mélanges de polymères contenant des groupes d'acide acrylique sous formation de polymères ou de copolymères hypoallergéniques dans une solution d'un amidon contenant un électrolyte, la solution étant composée de 5 à 50 pp de l'amidon, 10 à 50 pp du ou des monomères et 3 à 10 pp de l'électrolyte.

2. Gel selon la revendication 1, caractérise en ce qu'il peut être obtenu an utilisant de l'amylopectine comme amidon.

3. Gel selon les revendications 1 et 2, caractérisé en ce qu'il peut être obtenu en utilisant de l'acide acrylique ou méthacrylique, ou ses esters, ou des précurseurs de ces derniers.

4. Gel selon les revendications 1 à 3, caractérisé en ce qu'il peut être obtenu en utilisant une solution de l'amidon, laquelle contient un humectant.

5. Gel selon les revendications 1 à 4, caractérisé en que qu'il peut être obtenu en utilisant une solution de l'amidon, lequel amidon contient un substance qui augmente le pH.

6. Gel selon les revendications 1 à 5, caractérisé en ce qu'il peut être obtenu en utilisant une solution de l'amidon, laquelle contient un initiateur fournissent des radicaux libres ou un photoinitiateur pour la polymérisation des monomères.

7. Gel selon les revendications 1 à 6, caractérisé en ce qu'il peut être obtenu en utilisant une solution de l'amidon, dans laquelle est contenu en tant qu'électrolyte un sel hypoallergénique .

8. Gel selon les revendications 1 à 7, caractérisé en ce qu'il peut être obtenu en utilisant une solution aqueuse, dans laquelle est contenu, outre l'amidon, le ou les monomères et l'électrolyte, encore 1 à 2 pp d'un initiateur fournissant des radicaux libres.

9. Utilisation d'un gel selon les revendications 1 à 8 pour la confection d'électrodes à appliquer sur le corps humain.
